(19)

Europäisches Patentamt
European Patent Office
Office européen des brevets

(11) **EP 3 712 266 A1**

(12) **EUROPEAN PATENT APPLICATION**
published in accordance with Art. 153(4) EPC

(43) Date of publication:
**23.09.2020 Bulletin 2020/39**

(51) Int Cl.:
*C12N 15/09* (2006.01)    *A01K 67/027* (2006.01)
*C07K 16/46* (2006.01)    *C12N 5/078* (2010.01)

(21) Application number: **18878217.1**

(22) Date of filing: **19.11.2018**

(86) International application number:
**PCT/JP2018/042635**

(87) International publication number:
**WO 2019/098362 (23.05.2019 Gazette 2019/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.11.2017   JP 2017222215**

(71) Applicant: **University of Tsukuba**
**Tsukuba-shi, Ibaraki 305-8577 (JP)**

(72) Inventors:
• **TAKAHASHI Satoru**
  **Tsukuba-shi**
  **Ibaraki 305-8577 (JP)**
• **HAMADA Michito**
  **Tsukuba-shi**
  **Ibaraki 305-8577 (JP)**
• **JEON Hyojung**
  **Tsukuba-shi**
  **Ibaraki 305-8577 (JP)**

(74) Representative: **Cabinet Becker et Associés**
**25, rue Louis le Grand**
**75002 Paris (FR)**

(54) **NON-HUMAN ANIMAL AND METHOD FOR PRODUCING SAME**

(57)    A non-human animal is provided in which blood cells have a first genetic background, cells other than blood cells have a second genetic background, the first genetic background is different from the second genetic background, and the second genetic background is a genetic mutation that does not form hematopoietic stem cells.

**Description**

Technical Field

The present invention relates to a non-human animal and a method for producing the same. More specifically, the present invention relates to a non-human animal, a method for producing a human antibody, a method for producing a rat antibody, a method for producing human blood cells, and a method for producing a non-human animal in which a first genetic background of blood cells is different from a second genetic background of cells other than blood cells. Priority is claimed on Japanese Patent Application No. 2017-222215 filed on November 17, 2017, the content of which is incorporated herein by reference.

Background Art

Mice having human blood cells are known. These mice are prepared, for example, by destroying hematopoietic stem cells of immunodeficient mice such as NSG mice and NOD mice, and then transplanting human hematopoietic stem cells. Hematopoietic stem cell destruction is performed by radiation irradiation, administration of busulfan, which is an antineoplastic, and the like.

However, in some cases, mice having human blood cells prepared by a conventional method exhibited a poor engraftment rate of erythrocytes or lymphocytes.

Incidentally, for example, Non-Patent Literature 1 describes mice having a genetic mutation that does not form hematopoietic stem cells.

Citation List

Non-Patent Literature

[Non-Patent Literature 1]

Yokomizo T., et al., Characterization of GATA-1 + hemangioblastic cells in the mouse embryo, The EMBO Journal, 26, 184-196, 2007.

Summary of Invention

Technical Problem

An object of the present invention is to provide a new technique for producing a non-human animal in which a first genetic background of blood cells is different from a second genetic background of cells other than blood cells.

Solution to Problem

The present invention includes the following aspects.

[1] A non-human animal in which blood cells have a first genetic background, cells other than blood cells have a second genetic background, the first genetic background is different from the second genetic background, and the second genetic background is a genetic mutation that does not form hematopoietic stem cells.
[2] The non-human animal described in [1], in which substantially all blood cells have the first genetic background.
[3] The non-human animal described in [1] or [2], in which the second genetic background includes knockout of Runxl gene or myb gene, conditional knockout of Runxl gene or myb gene specific to hematopoietic stem cells, or conditional knockout of Runxl gene or myb gene specific to tissue involved in the development of hematopoietic stem cells.
[4] The non-human animal described in any one of [1] to [3], in which blood cells are human cells.
[5] The non-human animal described in any one of [1] to [3], in which blood cells are rat cells.
[6] A method for producing a human antibody specific to an antigen, including a step of immunizing the non-human animal described in [4] with the antigen.
[7] A method for producing a rat antibody specific to an antigen, including a step of immunizing the non-human animal described in [5] with the antigen.
[8] A method for producing human blood cells, including a step of collecting blood cells from the non-human animal described in [4].

[9] A method for producing a non-human animal in which a first genetic background of blood cells is different from a second genetic background of cells other than blood cells, the method including a step of transplacentally transplanting hematopoietic stem cells having a first genetic background into an early embryo of a non-human animal having a second genetic background, and a step of growing the early embryo so as to obtain a non-human animal having hematopoietic stem cells, in which the second genetic background is a genetic mutation that does not form hematopoietic stem cells, and the first genetic background is different from the second genetic background.

[10] The method for producing described in [9], in which the second genetic background includes knockout of Runxl gene or myb gene, conditional knockout of Runxl gene or myb gene specific to hematopoietic stem cells, or conditional knockout of Runxl gene or myb gene specific to tissue involved in the development of hematopoietic stem cells.

[11] The method for producing described in [9] or [10], in which the hematopoietic stem cells are human cells.

[12] The method for producing described in [9] or [10], in which the hematopoietic stem cells are rat cells.

Advantageous Effects of Invention

According to the present invention, it is possible to provide a new technique for producing a non-human animal in which a first genetic background of blood cells is different from a second genetic background of cells other than blood cells.

Brief Description of Drawings

Fig. 1 is a schematic diagram illustrating a method for producing a non-human animal according to one embodiment.

Figs. 2(a) to 2(c) show photographs of mouse fetuses in Experimental Example 2 and graphs showing the typical results of flow cytometry analysis. Fig. 2(d) is a graph obtained by plotting a chimerism of wild-type mice and rescued Runx1-/-::Tg mice in Experimental Example 2.

Figs. 3(a) and 3(b) show graphs of the results of colony assay in Experimental Example 2. Fig. 3(c) shows a graph of the results of measuring a chimerism of blood cell colonies in Experimental Example 2.

Fig. 4 shows graphs of the results of flow cytometry analysis on liver cells in Experimental Example 2.

Fig. 5 shows graphs of the results of flow cytometry analysis on spleen cells in Experimental Example 2.

Fig. 6(a) is a photograph of a fetus of a wild-type mouse, and Fig. 6(b) is a photograph of a fetus of Runx1$^{-/-}$::Tg mouse rescued by the transplantation of rat hematopoietic stem cells in Experimental Example 3.

Fig. 7(a) shows a graph of the typical results of flow cytometry analysis in Experimental Example 4. Fig. 7(b) is a graph obtained by plotting a chimerism of wild-type mice and rescued Runx1$^{-/-}$::Tg mice in Experimental Example 4.

Fig. 8 shows graphs of the results of flow cytometry analysis on liver cells in Experimental Example 4.

Figs. 9(a) and 9(b) show graphs of the typical results of flow cytometry analysis in Experimental Example 5.

Fig. 10 shows a graph of the typical results of flow cytometry analysis in Experimental Example 6.

Figs. 11(a) and 11(b) show graphs of the typical results of flow cytometry analysis in Experimental Example 7.

Figs. 12(a) to 12(c) show graphs of the results of flow cytometry analysis in Experimental Example 8.

Fig. 13 is a photograph showing the results of Western blotting in Experimental Example 9.

Description of Embodiments

[Non-human animal]

In one embodiment, the present invention provides a non-human animal in which blood cells have a first genetic background, cells other than blood cells have a second genetic background, the first genetic background is different from the second genetic background, and the second genetic background is a genetic mutation that does not form hematopoietic stem cells.

As will be described later in the examples, in the non-human animal of the present embodiment, blood cells have the first genetic background, cells other than blood cells have the second genetic background, and the first genetic background is different from the second genetic background.

That is, the non-human animal of the present embodiment is obtained by replacing blood cells of a non-human animal which originally has the second genetic background with cells having the first genetic background.

In the present specification, blood cells mean all the cells differentiated from hematopoietic stem cells. Therefore, in the present specification, blood cells mean leukocytes (neutrophils, eosinophils, basophils, lymphocytes, monocytes, and macrophages), erythrocytes, platelets, mast cells, dendritic cells, and the like.

In the non-human animal of the present embodiment, the engraftment rate of erythrocytes and lymphocytes having the first genetic background is high, and the immune system is constructed by blood cells having the first genetic background.

The non-human animal is not particularly limited, and examples thereof include mice, rats, rabbits, pigs, sheep, goats, cows, monkeys, and the like.

In the non-human animal of the present embodiment, cells having different genetic background mean cells of different species or allogenic cells of the same species. That is, cells having different genetic background mean cells having different types of major histocompatibility complex antigens, cells from congenic strains, and the like.

In the non-human animal of the present embodiment, the second genetic background is a genetic mutation that does not form hematopoietic stem cells. The genetic mutation that does not form hematopoietic stem cells means a genetic mutation that does not form hematopoietic stem cells due to the mutation or deletion of a specific gene. Specifically, examples thereof include Run-related transcription factor 1 $(Runx1)^{-/-}$, $myb^{-/-}$, and the like.

The human Runxl protein has a plurality of isoforms assigned with NCBI accession numbers such as NP_001116079.1, NP_001001890.1, and NP_001745.2. The NCBI accession number of the mouse Runxl protein is NP_001104491.1 or the like.

Furthermore, the human myb protein has a plurality of isoforms assigned with the NCBI accession numbers such as NP_001123645.1, NP_001155129.1, and NP_001155130.1. The NCBI accession number of the mouse myb protein is NP_001185843.1 or the like.

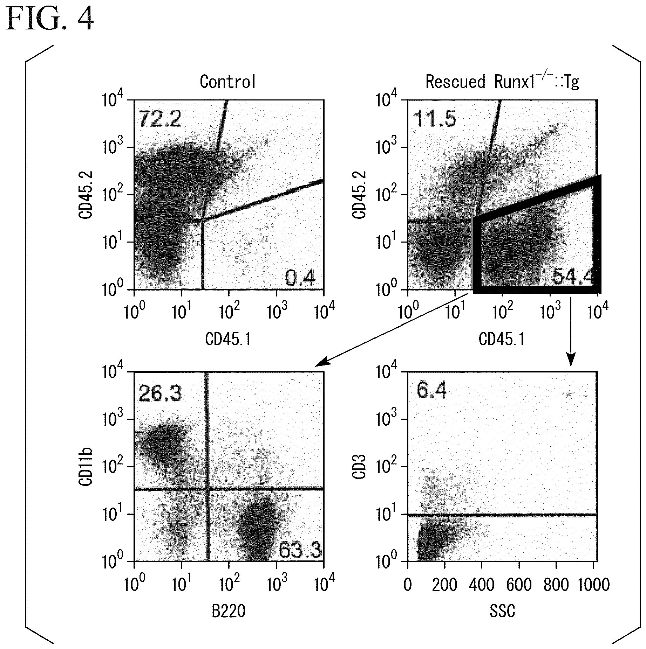

For example, a $Runx1^{-/-}$ mouse is known to die at around embryonic day 12.5 due to the lack of adult hematopoiesis in the fetal liver. In contrast, as described in Non-Patent Literature 1, for example, a $Runx1^{-/-}$ mouse $(Runx1^{-/-}$::G1-HRD-Runx1), into which Runx1 cDNA (G1-HRD-Runx1) linked to the downstream of a GATA-1 hematopoietic regulatory domain is introduced as a transgene, does not form hematopoietic stem cells but grows until birth. Here, "::" means having a transgene. An example of the G1-HRD-Runx1 construct is described in, for example, Fig. 4A of Non-Patent Literature 1 and the like.

As long as the second genetic background in the non-human animal of the present embodiment does not form hematopoietic stem cells, the second genetic background may be, for example, a genetic mutation or genetic modification of the aforementioned $Runx1^{-/-}$::G1-HRD-Runx1 and the like.

In addition, as described above, the $Runx1^{-/-}$::G1-HRD-Runx1 mouse grows until birth. However, the $Runx1^{-/-}$::G1-HRD-Runx1 mouse dies within several hours after birth. It is considered that this is because Runxl is also involved in the development of the nervous system or sternum.

Therefore, as the second genetic background, for example, a genetic mutation or genetic modification, such as $Runx1^{f/f}$::Tie2-Cre::G1-HRD-Runx1, or $Runx1^{f/-}$::Tie2-Cre::G1-HRD-Runx1, may be used which results in a non-human animal in which hematopoietic stem cells are not formed but other genetic traits are substantially normal. Such a non-human animal can be grown normally by being transplanted with functional hematopoietic stem cells. Herein, the functional hematopoietic stem cells may be hematopoietic stem cells having a normal (wild-type) genetic background or a genetic background with at least a normal hematologic system. Tie2 is a receptor tyrosine kinase that is expressed in the precursor cells common to the vascular endothelium and blood cells in the early embryonic period. One example of the Tie2-Cre construct is described in, for example, Kisanuki Y.Y., Tie2-Cre Transgenic Mice: A New Model for Endothelial Cell-Lineage Analysis in Vivo, Developmental Biology 230, 230-242, 2001., and the like.

Herein, "$Runx1^{f/f}$" means that at least a part of the exon has the Runx1 gene interposed between two loxP sequences in a homologous region. "$Runx1^{f/-}$" means that at least a part of the exon has the Runx1 gene interposed between two loxP sequences in one of the genomes but does not have the Runxl gene in the other genome. Furthermore, Tie2-Cre means that the Tie2 gene has a transgene in which a Cre recombinase gene is linked to the downstream of the promoter of the Tie2 gene. The Tie2 gene is a gene expressed in hematopoietic stem cells, vascular endothelial cells involved in the development of hematopoietic stem cells, and the like. Therefore, in a non-human animal having a genetic modification such as $Runx1^{f/f}$::Tie2-Cre or $Runx1^{f/-}$::Tie2-Cre, the Runxl gene is conditionally deleted in the hematopoietic stem cells or the vascular endothelial cells involved in the development of hematopoietic stem cells. As a result, a phenotype that does not form hematopoietic stem cells is obtained.

That is, the second genetic background in the non-human animal of the present embodiment may include the knockout of the Runxl gene or the myb gene, the conditional knockout of the Runxl gene or the myb gene specific to hematopoietic stem cells, or the conditional knockout of the Runxl gene or the myb gene specific to the tissue involved in the development of hematopoietic stem cells.

Herein, "include" means that the second genetic background in the non-human animal of the present embodiment may have the aforementioned genetic mutation that does not form hematopoietic stem cells and a genetic modification such as "::G1-HRD-Runxl".

In the non-human animal of the present embodiment, it is preferable that the second genetic background be not a genetic mutation that causes immunodeficiency. Examples of the genetic background that causes immunodeficiency include NOD. $Cg$-$Prkdc^{scid}II2rg^{tm1Wjl}$/SzJ (the same genetic background as that of the NSG mouse), NOD/Shi-scid-$IL2R\gamma^{null}$ (the same genetic background as that of the NOG mouse), and the like. In addition, it is preferable that the non-human animal of the present embodiment not experience the destruction of hematopoietic stem cells by the radiation irradiation, the administration of busulfan, and the like.

The first genetic background in the non-human animal of the present embodiment may be a normal (wild-type) genetic background or a genetic background with at least a normal hematologic system. Blood cells having the first genetic background may be, for example, blood cells of a species different from that of the non-human animal of the present embodiment, allogenic blood cells of the same species as that of the non-human animal of the present embodiment, or congenic blood cells of the same species as that of the non-human animal of the present embodiment. More specifically, for example, the non-human animal may be a mouse, and blood cells having the first genetic background may be human blood cells, rat blood cells, congenic mouse blood cells, and the like.

In the non-human animal of the present embodiment, it is preferable that substantially all blood cells have the first genetic background. In a case where substantially all blood cells have the first genetic background, blood cells of the non-human animal of the present embodiment, which originally have the second genetic background, are totally replaced with the cells having the first genetic background.

Herein, "substantially all blood cells have the first genetic background" means that the proportion of blood cells having the first genetic background is equal to or higher than 80%, preferably equal to or higher than 90%, even more preferably equal to or higher than 95%, and particularly preferably 100%.

In the non-human animal of the present embodiment, blood cells having the first genetic background may be human cells or rat cells. For example, the non-human animal of the present embodiment may be a mouse, a rabbit, a pig, a sheep, a goat, a cow, or the like having human cells or rat cells as blood cells.

In a case where the non-human animal of the present embodiment has the human hematologic system, the non-human animal can be used as a human disease model or the like. Alternatively, the non-human animal can be used for drug efficacy evaluation in drug development, drug screening, and the like.

[Method for producing human antibody]

In one embodiment, the present invention provides a method for producing a human antibody specific to an antigen, including a step of immunizing the aforementioned non-human animal having human cells as blood cells with the antigen.

As the non-human animal, for example, a mouse is suitable. That is, according to the production method of the present embodiment, an antigen-specific human monoclonal antibody can be easily produced by immunizing a mouse having human cells as blood cells with an antigen.

Conventional methods can be used as the method for immunizing the mouse with an antigen. In addition, as a method for producing the antibody, a conventional method for producing a hybridoma can be used.

The human monoclonal antibody obtained by the method of the present embodiment is a fully human antibody. Therefore, this antibody is less likely to cause side effects such anaphylactic shock even when administered to humans, and can be used as an antibody drug.

In a case where a bigger animal such as a rabbit is used as the non-human animal, a polyclonal antibody can be produced. That is, an antigen-specific human polyclonal antibody can be produced by immunizing a rabbit having human cells as blood cells with an antigen.

[Method for producing rat antibody]

In one embodiment, the present invention provides a method for producing a rat antibody specific to an antigen, including a step of immunizing the aforementioned non-human animal having rat cells as blood cells with the antigen.

As the non-human animal, for example, a mouse is suitable. That is, according to the production method of the present embodiment, an antigen-specific rat monoclonal antibody can be easily produced by immunizing a mouse having rat cells as blood cells with an antigen.

Conventionally, rat monoclonal antibodies have been prepared by immunizing a rat with an antigen. However, sometimes it is easier to handle a mouse than to handle a rat. Therefore, as long as a rat monoclonal antibody can be produced using a mouse, sometimes a rat monoclonal antibody can be prepared more easily, which is convenient.

Conventional methods can be used as the method for immunizing the mouse with an antigen. In addition, as a method for producing the antibody, a conventional method for producing a hybridoma can be used.

In a case where a bigger animal such as a rabbit is used as the non-human animal, a polyclonal antibody can be produced. That is, an antigen-specific rat polyclonal antibody can be produced by immunizing a rabbit having rat cells as blood cells with an antigen.

[Method for producing human blood cells]

In one embodiment, the present invention provides a method for producing human blood cells, including a step of collecting blood cells from the aforementioned non-human animal having human cells as blood cells.

Currently, blood for transfusion cannot be produced artificially, and it is difficult to store blood for a long period of time. Therefore, blood for transfusion is secured by blood donation. However, it has been pointed out that a stable supply of blood may become difficult due to a decrease in blood donors resulting from the low birthrate and aging population.

On the other hand, according to the production method of the present embodiment, it is possible to industrially produce blood cells such as erythrocytes and platelets, and to use these for producing blood products for transfusion.

In the production method of the present embodiment, as the non-human animal, for example, pigs, sheep, goats, cows, and the like can be suitably used. That is, according to the production method of the present embodiment, by collecting blood cells from the non-human animal having human cells as blood cells, it is possible to stably produce human blood cells.

[Method for producing non-human animal]

In one embodiment, the present invention provides a method for producing a non-human animal in which a first genetic background of blood cells is different from a second genetic background of cells other than blood cells, the method including a step of transplacentally transplanting hematopoietic stem cells having a first genetic background into an early embryo of a non-human animal having a second genetic background, and a step of growing the early embryo so as to obtain hematopoietic stem cells, wherein the second genetic background is a genetic mutation that does not form hematopoietic stem cells, and the first genetic background is different from the second genetic background.

The aforementioned non-human animal can be produced by the production method of the present embodiment. In the production method of the present embodiment, the non-human animal, the first genetic background, and the second genetic background are the same as those described above.

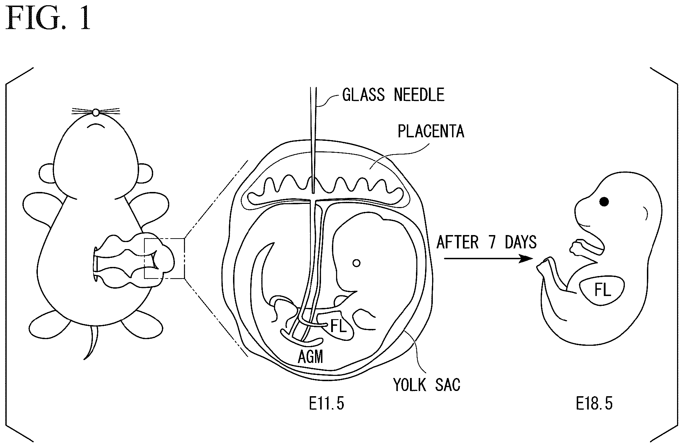

Fig. 1 is a schematic diagram illustrating an example of the production method of the present embodiment. In the example shown in Fig. 1, the non-human animal is a mouse.

First, a mother mouse with an early embryo (fetus) having a genetic mutation that does not form hematopoietic stem cells is anesthetized, and the mother's abdomen is incised to expose the uterus. Herein, depending on the genetic background of the mother mouse used, sometimes the mother mouse has both the fetus having a genetic background forming hematopoietic stem cells and the fetus having a genetic background not forming hematopoietic stem cells.

The genetic mutation that does not form hematopoietic stem cells is as described above, and examples thereof include the knockout of the Runxl gene or the myb gene, the conditional knockout of the Runxl gene or the myb gene specific to hematopoietic stem cells, the conditional knockout of the Runxl gene or the myb gene specific to tissue involved in the development of hematopoietic stem cells, and the like.

More specifically, examples thereof include, but are not limited to, genetic mutations such as Runx1$^{-/-}$::G1-HRD-Runx1, Runx1$^{f/f}$::Tie2-Cre::G1-HRD-Runx1, and Runx1$^{f/-}$::Tie2-Cre::G1-HRD-Runx1.

The above fetus is preferably a fetus at a developmental stage corresponding to the period during which hematopoietic stem cells develop in a wild-type fetus. For example, in the case of a mouse, it is preferably a fetus on embryonic day 10 to 11.

Subsequently, desired hematopoietic stem cells are injected by inserting a needle into the placenta from the outside of the exposed uterus, such that the hematopoietic stem cells are transplacentally transplanted into the fetus. The number of hematopoietic stem cells to be transplanted may be one or more. Furthermore, the hematopoietic stem cells may be transplanted after being purified, or a cell population containing the hematopoietic stem cells may be transplanted. Examples of the cell population containing the hematopoietic stem cells include, but are not limited to, cells derived from the fetal liver, bone marrow cells derived from a living body, and the like.

Herein, examples of the needle include an injection needle, a glass needle, and the like. Among these, a glass needle is preferable, and a glass needle with a polished tip is particularly preferable. The tip of the glass needle can be polished using, for example, a polishing machine (model "EG-4", NARISHIGE Group.) and the like. The thickness of the tip of the glass needle is preferably about 50 to 62.5 $\mu$m. The inventors of the present invention have found that by polishing the tip of the glass needle, the success rate of transplantation is dramatically improved.

The hematopoietic stem cells to be transplanted may be hematopoietic stem cells at a developmental stage corresponding to hematopoietic stem cells at an early stage of development, hematopoietic stem cells at a later stage of development, or adult-derived hematopoietic stem cells. For example, hematopoietic stem cells generated from pluripotent stem cells by the induction of differentiation may be used. The pluripotent stem cells are not particularly limited, and examples thereof include embryonic stem cells (ES cells), iPS cells, and the like.

The inventors of the present invention have succeeded in obtaining a mouse having blood cells derived from the transplanted hematopoietic stem cells, regardless of the stage of development of the hematopoietic stem cells to be transplanted.

The hematopoietic stem cells to be transplanted may be, for example, human cells or rat cells.

After the transplantation of hematopoietic stem cells, the uterus is returned to the mother mouse, the skin is sutured, and the fetuses are allowed to grow. Fetuses that failed to be transplanted successfully with the hematopoietic stem cells died due to the deficiency of hematopoietic stem cells. On the other hand, fetuses successfully transplanted with the hematopoietic stem cells and those having a genetic background forming hematopoietic stem cells were born on embryonic day 19. The grown fetuses may be extracted by cesarean.

The grown fetuses include a non-human animal in which the first genetic background of blood cells is different from the second genetic background of cells other than blood cells, and the second genetic background is a genetic mutation that does not form hematopoietic stem cells.

For example, in a case where cells other than blood cells among the somatic cells of a fetus are found to have the second genetic background as a result of the genomic DNA analysis, the fetus is a non-human animal of interest, that is, a non-human animal in which the first genetic background of blood cells is different from the second genetic background of cells other than blood cells.

Examples

Next, the present invention will be more specifically described based on examples, but the present invention is not limited to the following examples.

[Experimental example 1]

(Transplantation 1 of mouse hematopoietic stem cells)

«Examination on transhepatic transplantation»

Fetuses in a mother Ly5.2 mouse (hereinafter, referred to as "Runx1$^{+/-}$::Tg mouse" in some cases) having the genotype of Runx1$^{+/-}$::G1-HRD-Runx1 were transplanted with hematopoietic stem cells of a C57BL/6-Ly5.1 mouse (hereinafter, referred to as "donor mouse" in some cases) as a congenic mouse.

As the transplantation method, hematopoietic stem cells of the donor mouse were transplanted into a fetus on embryonic day 13.5 or embryonic day 14.5 by using an injection needle (30 gauge, Terumo Corporation) through the liver. Specifically, each of the fetuses was transplanted with 1 $\mu$L of liver cells of a fetus of the donor mouse on embryonic day 14.5 at a cell density adjusted to $2 \times 10^5$ cells/$\mu$L.

Although the transplantation was performed on 232 fetuses, a Runx1$^{-/-}$::Tg mouse rescued by the hematopoietic stem cells from the donor mouse was not obtained at all.

From this result, it was considered that the fetus was likely to be damaged by the transhepatic transplantation operation.

<<Examination on transplacental transplantation>>

Subsequently, transplacental transplantation was examined. Specifically, fetuses on embryonic day 11.5 of a mother Runx1$^{+/-}$::Tg mouse were transplacentally transplanted with the hematopoietic stem cells of the donor mouse.

First, the mother Runx1$^{+/-}$::Tg mouse was anesthetized with isoflurane, and the abdomen was incised to expose the uterus. Subsequently, a glass needle was inserted into the placenta, such that the hematopoietic stem cells of the donor mouse were transplanted.

As the glass needle, a glass needle with tip polished using a polishing machine (model "EG-4", NARISHIGE Group) was used. The thickness of the tip was about 50 $\mu$m.

As the hematopoietic stem cells, 1 $\mu$L of liver cells of a fetus on embryonic day 14.5 of the donor mouse were transplanted into each fetus at a cell density adjusted to $2 \times 10^5$ cells/$\mu$L.

After the transplantation of the hematopoietic stem cells, the uterus was returned to the mother mouse, the skin was sutured, and the fetuses were allowed to grow. Seven days after the transplantation of the hematopoietic stem cells, fetuses on embryonic day 18.5 were extracted by cesarean and analyzed.

As a result of transplanting the hematopoietic stem cells into 401 fetuses, 267 mice survived until embryonic day 18.5. The survival rate was about 66.6%. The Runx1$^{-/-}$::Tg mice rescued by the hematopoietic stem cells from the donor mouse appeared normal. Accordingly, it was considered that the hematopoietic system was successfully reconstituted.

[Experimental example 2]

(Analysis of mice subjected to transplantation)

The Runx1$^{-/-}$::Tg mice rescued by the transplacental transplantation of the hematopoietic stem cells from the donor mouse in Experimental Example 1 were more specifically analyzed.

<<Examination on chimerism>>

First, by flow cytometry analysis using an anti-CD45.1 antibody and an anti-CD45.2 antibody, a chimerism of donor mouse-derived cells in the liver cells of the rescued Runx1$^{-/-}$::Tg mice was examined. The chimerism of the donor mouse-derived cells was calculated by the following Equation (1).

$$\text{Chimerism } (\%) = (\text{number of cells positive for CD45.1/(number of cells positive}$$

$$\text{for CD45.1 + number of cells positive for CD45.2)}) \times 100 \cdots (1)$$

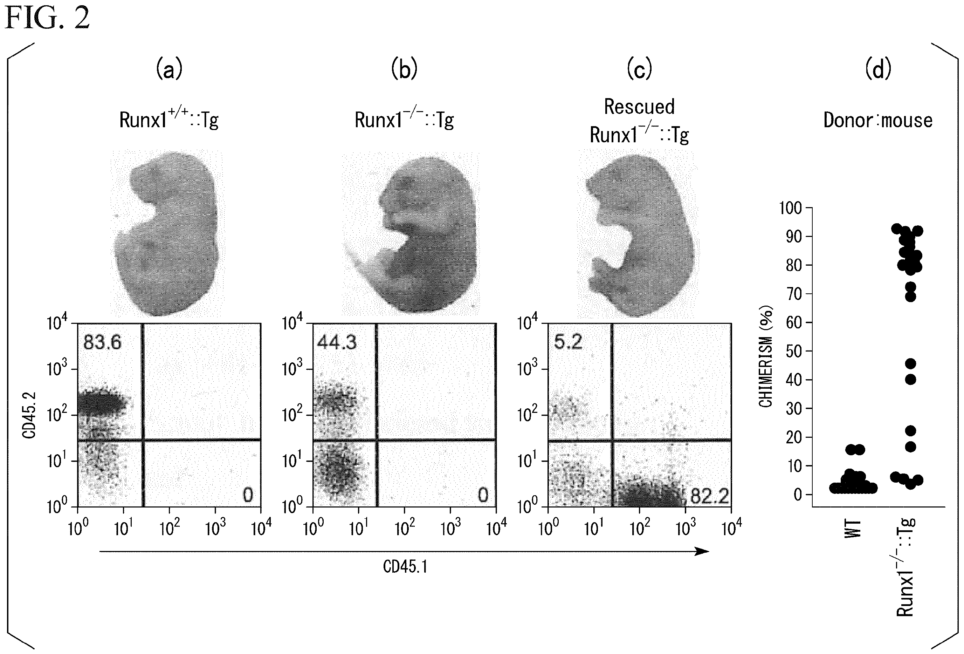

Figs. 2(a) to 2(c) show photographs of fetuses and graphs showing the typical results of the flow cytometry analysis. Fig. 2(a) shows a typical photograph of a Runx1$^{+/+}$::Tg mouse and a graph showing the analysis results. Fig. 2(b) shows a typical photograph of a Runx1$^{-/-}$::Tg mouse that was not rescued and a graph showing the analysis results. Fig. 2(c) shows a typical photograph of a rescued Runx1$^{-/-}$::Tg mouse and a graph showing the analysis results. Fig. 2 (d) is a graph obtained by plotting the chimerism of wild-type mice (WT) and rescued Runx1$^{-/-}$::Tg mice. In Fig. 2(d), "Donor:mouse" means that the donor of the hematopoietic stem cells is a mouse.

As a result, among 32 Runx1$^{-/-}$::Tg mice transplanted with the hematopoietic stem cells, 23 Runx1$^{-/-}$::Tg mice showed a chimerism equal to or higher than 0.4%. In contrast, it was revealed that the wild-type mice transplanted with the hematopoietic stem cells from the donor mouse show only a low chimerism.

Those showing a chimerism lower than 100% were considered to show such a rate because the chimerism was analyzed on embryonic day 18.5. Presumably, in a case where the fetuses were allowed to grow for a longer period of time, the chimerism may become close to 100%. However, because the Runx1$^{-/-}$::Tg mice died shortly after birth, in order to make the mice grow for a longer period of time, it is necessary to perform analysis by using a mouse having a genotype such as Runx1$^{f/f}$::Tie2-Cre::G1-HRD-Runx1 or Runx1$^{f/-}$::Tie2-Cre::G1-HRD-Runx1.

<<Examination on CFU>>

Subsequently, by using each of the Runx1$^{+/+}$::Tg mice, the unrescued Runx1$^{-/-}$::Tg mice, and the rescued Runx1$^{-/-}$::Tg mice on embryonic day 18.5, the colony assay was performed on fetal liver cells.

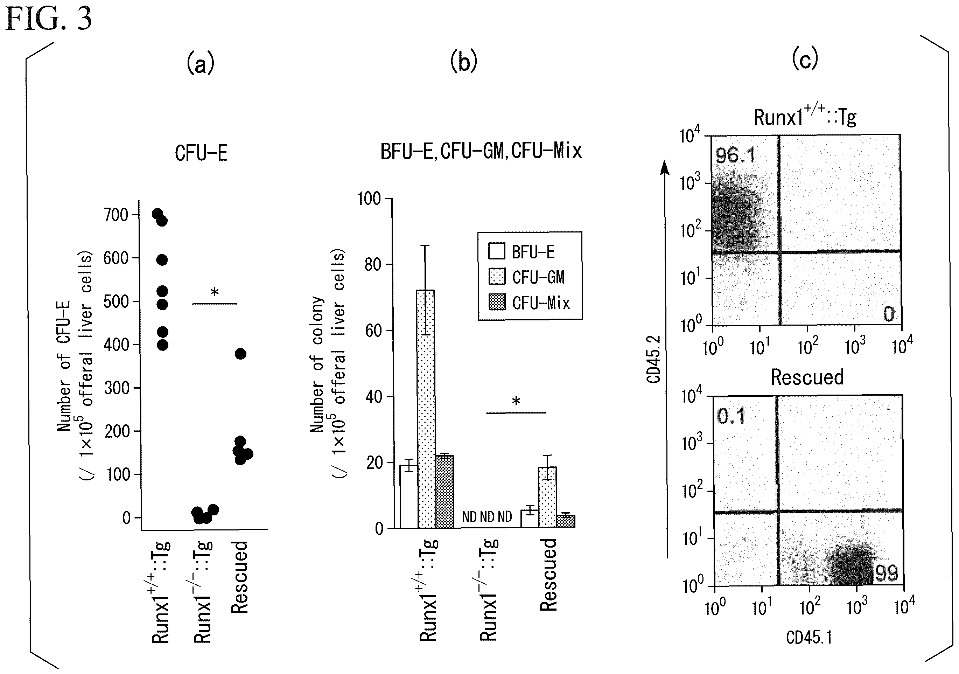

Figs. 3(a) and 3(b) show graphs of the results of the colony assay. Fig. 3 (a) shows the number of erythroid colony-forming units (CFU-E) per $1 \times 10^5$ fetal liver cells, and Fig. 3 (b) shows the number of erythroid burst forming units (BFU-E), granulocyte macrophage colony-forming units (CFU-GM), and mixed colony-forming units (CFU-Mix) including myeloid cells, erythrocytes, and megakaryocytes per $1 \times 10^5$ fetal liver cells.

In Figs. 3(a) and 3(b), "Runx1$^{-/-}$::Tg" means the result obtained from the unrescued Runx1$^{-/-}$::Tg mice, and "Rescued" means the result obtained from the rescued Runx1$^{-/-}$::Tg mice. Furthermore, in Fig. 3(b), "ND" means that no colony was detected.

As a result, it was revealed that the colony-forming units CFU-E, BFU-E, CFU-GM, and CFU-Mix were detected in the fetal liver cells of the rescued Runx1$^{-/-}$::Tg mice but were not detected in the fetal liver cells of the unrescued Runx1$^{-/-}$::Tg mice.

Fig. 3(c) shows the results of measuring the chimerism of the aforementioned colonies derived from the Runx1$^{+/+}$::Tg mice and the rescued Runx1$^{-/-}$::Tg mice by the flow cytometry analysis using the anti-CD45.1 antibody and the anti-CD45.2 antibody. In Fig. 3(c), "Rescued" means the results obtained from the rescued Runx1$^{-/-}$::Tg mice.

As a result, it was revealed that the aforementioned colonies derived from the rescued Runx1$^{-/-}$::Tg mice were constituted with the cells derived from the donor mouse positive for CD45.1 with high chimerism.

<<Examination on liver cells and spleen cells>>

Subsequently, whether or not the donor mouse-derived cells in the liver and spleen of the rescued Runx1$^{-/-}$::Tg mouse were differentiated into macrophages, B lymphocytes, and T lymphocytes was analyzed by flow cytometry.

The CD11b antigen was detected as a macrophage marker. The B220 antigen was detected as a B lymphocyte marker. The CD3 antigen was detected as a T lymphocyte marker. Fig. 4 shows graphs of the results of the flow cytometry analysis on liver cells.

As a result, it was revealed that the donor mouse-derived cells in the liver of the rescued Runx1[-/-]::Tg mice are differentiated into macrophages, B lymphocytes, and T lymphocytes.

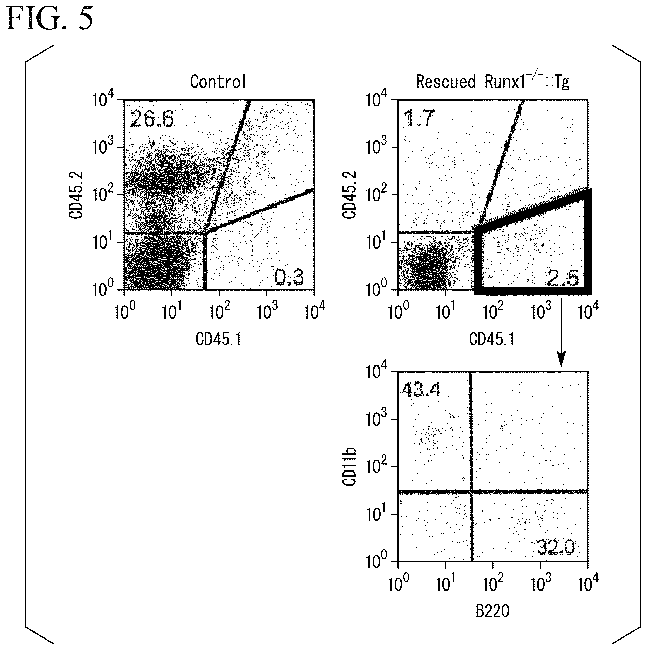

Fig. 5 shows graphs of the results of the flow cytometry analysis on spleen cells. As a result, it was revealed that although the number of donor mouse-derived cells in the spleen is small, at least macrophages and B lymphocytes are present in the spleen.

[Experimental example 3]

(Transplantation of rat hematopoietic stem cells)

Whether or not the Runx1[-/-]::Tg mice can be rescued by heterologous hematopoietic stem cells was examined.

Specifically, fetuses of a mother Runx1[+/-]::Tg mouse were transplacentally transplanted with rat hematopoietic stem cells in the same manner as in Experimental Example 1, except that liver cells of a rat fetus on embryonic day 15.5 were used as cells to be transplanted.

As a result of transplanting the hematopoietic stem cells into 53 fetuses, 36 living fetuses were obtained. Among the 36 living fetuses, 5 fetuses were Runx1[-/-]::Tg mice. Among these, 4 fetuses appeared normal. Therefore, it was considered that the hematopoietic system was successfully reconstituted by the rat hematopoietic stem cells.

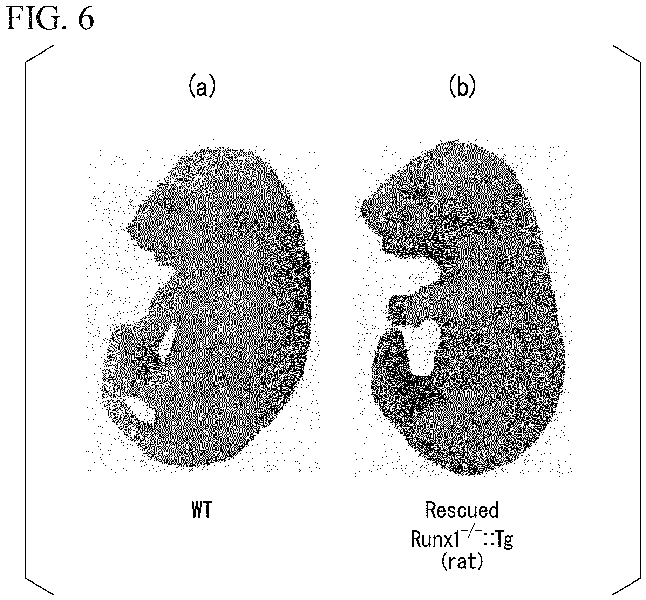

Fig. 6(a) is a photograph of a fetus of a wild-type mouse, and Fig. 6(b) is a photograph of a fetus of a Runx1[-/-]::Tg mouse rescued by the transplantation of the rat hematopoietic stem cells.

[Experimental example 4]

(Analysis of mice subjected to transplantation)

The Runx1[-/-]::Tg mice rescued by the transplacental transplantation of the rat hematopoietic stem cells in Experimental Example 3 were more specifically analyzed.

<<Examination on chimerism>>

First, by flow cytometry analysis using an anti-mouse anti-CD45.2 antibody and an anti-rat CD45 antibody, a chimerism of rat-derived cells in the liver cells of the rescued Runx1[-/-]::Tg mice on embryonic day 18.5 was examined. The chimerism of the rat-derived cells was calculated by the following Equation (2).

$$\text{Chimerism } (\%) = (\text{number of cells positive for rat CD45}/(\text{number of cells}$$

$$\text{positive for rat CD45} + \text{number of cells positive for mouse CD45.2})) \times 100 \cdots (2)$$

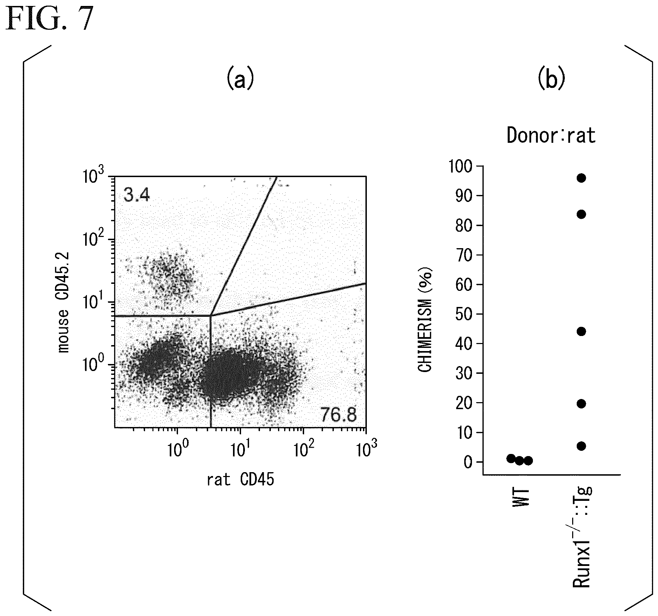

Fig. 7(a) shows a graph of the typical results of the flow cytometry analysis. Fig. 7(b) is a graph obtained by plotting the chimerism of wild-type mice (WT) and the rescued Runx1[-/-]::Tg mice. In Fig. 7(b), "Donor:rat" means that the donor of the hematopoietic stem cells is a rat.

As a result, it was revealed that the Runx1[-/-]::Tg mice transplanted with the rat hematopoietic stem cells exhibited a chimerism of up to 96%. In contrast, it was revealed that the wild-type mice transplanted with the rat hematopoietic stem cells exhibited only a low chimerism.

<<Examination on liver cells>>

Subsequently, whether or not the rat-derived cells in the liver of the rescued Runx1[-/-]::Tg mice showing a chimerism of 96% are differentiated into macrophages, B lymphocytes, T lymphocytes, and erythrocytes was analyzed by flow cytometry.

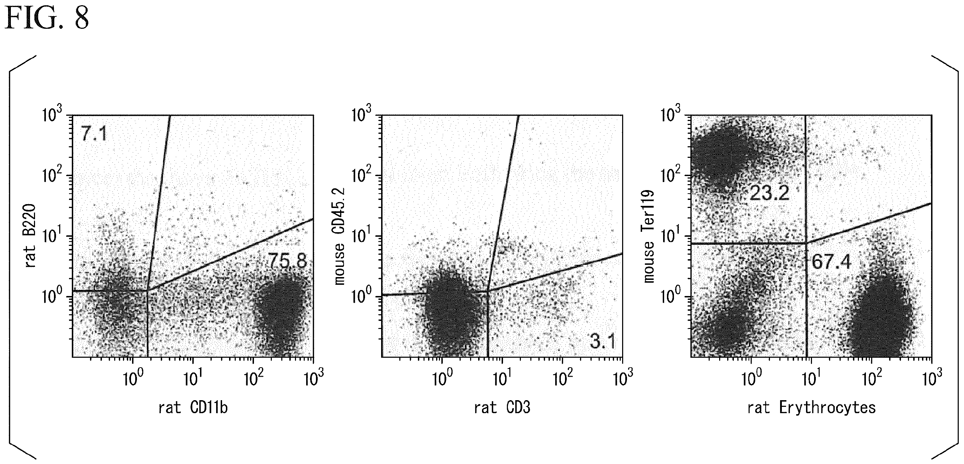

The rat CD11b antigen was detected as a macrophage marker. The rat B220 antigen was detected as a B lymphocyte marker. The rat CD3 antigen was detected as a T lymphocyte marker. The rat Ter119 antigen and the mouse Ter119 antigen were detected as erythrocyte markers. Fig. 8 shows a graph of the results of the flow cytometry analysis.

As a result, it was revealed that the rat-derived cells in the liver of the rescued Runx1[-/-]::Tg mice are differentiated into macrophages, B lymphocytes, T lymphocytes, and erythrocytes.

Furthermore, it was revealed that there were a large number of rat erythrocytes in the liver of the rescued Runx1-/-::Tg mice. Specifically, the proportion of the rat erythrocytes was 67.4%, and the proportion of the mouse erythrocytes was 23.2%.

From the above results, it was revealed that the transplacental transplantation makes it possible to rescue the Runx1$^{-/-}$::Tg mice by using heterologous hematopoietic stem cells.

[Experimental example 5]

(Transplantation 1 of human hematopoietic stem cells)

Whether or not the Runx1$^{-/-}$::Tg mice can be rescued by human hematopoietic stem cells was examined.

<<Culturing of human hematopoietic stem cells>>

With reference to the prior art, hematopoietic stem cells derived from human cord blood were cultured (see Boitano, A. E et al., Aryl hydrocarbon receptor antagonists promote the expansion of human hematopoietic stem cells, Science, 329 (5997), 1345-1348, 2010. ; Wagner, JE, Jr. et al., Phase I/II Trial of StemRegenin-1 Expanded Umbilical Cord Blood Hematopoietic Stem Cells Supports Testing as a Stand-Alone Graft, Stem Cell, 18 (1), 144-155, 2016.) The hematopoietic stem cells derived from human cord blood were provided from Professor Shigeru Chiba, Department of Hematology, University of Tsukuba.

Specifically, by using a medium obtained by adding human thrombopoietin, human interleukin (IL)-6, human Fms-related tyrosine kinase 3 ligand (Flt3-L), a human stem cell factor (all manufactured by R&D Systems), and 750 nM StemRegenin 1, STEMCELL Technologies at 100 ng/mL to a serum-free medium for growing human cord blood-derived hematopoietic stem cells (trade name "StemSpan SFEM", STEMCELL Technologies), human cord blood-derived hematopoietic stem cells at $5 \times 10^4$ cells/mL positive for CD34 were cultured in a 6-well plate. The cells were cultured under the conditions of 5% CO2 and 37°C. The cord blood-derived hematopoietic stem cells cultured for one week or longer were collected and used for transplantation experiments.

<<Transplacental transplantation>>

On embryonic day 11.5, each of the fetuses of a Runx1$^{+/-}$:: Tg mother mouse was transplacentally transplanted with 20 ng of human cytokines (thrombopoietin, IL-6, Flt3-L, and stem cell factor) and $1 \times 10^4$ human cord blood-derived hematopoietic stem cells in the same manner as in Experimental Example 1. Then, on embryonic day 18.5, the livers of the fetuses were collected and analyzed by flow cytometry.

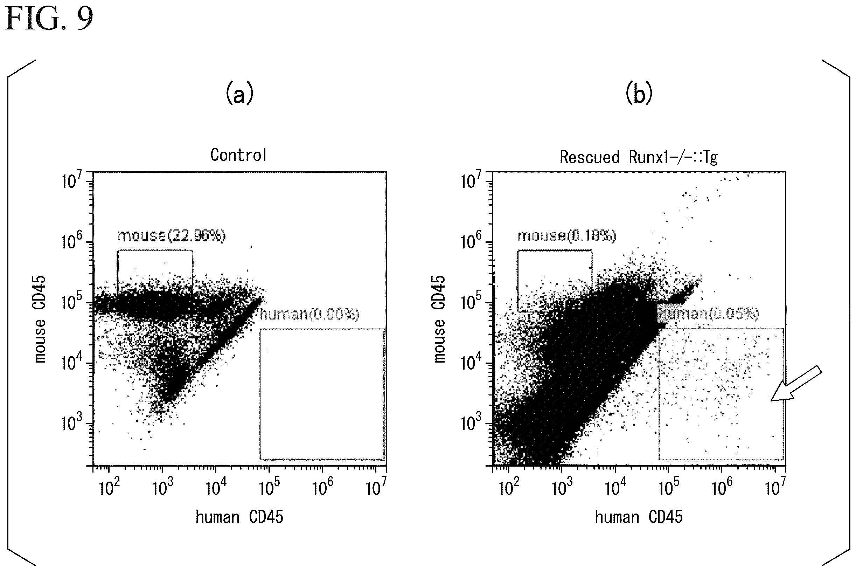

Figs. 9(a) and 9(b) show graphs of the typical results of the flow cytometry analysis. Fig. 9(a) shows the results obtained from the fetuses into which the human hematopoietic stem cells were not transplanted, and Fig. 9(b) shows the results obtained from the fetuses of the Runx1$^{-/-}$::Tg mouse rescued by the transplantation of the human hematopoietic stem cells. In Fig. 9(b), the arrow indicates the cells positive for human CD45.

As a result, the cells positive for human CD45 were detected in the rescued Runx1$^{-/-}$::Tg mice. Therefore, it was confirmed that the human-derived cells survive in the mouse fetus.

[Experimental example 6]

(Transplantation 2 of human hematopoietic stem cells)

Whether or not wild-type mice can be rescued by human hematopoietic stem cells was examined. First, human cord blood-derived hematopoietic stem cells were prepared in the same manner as in Experimental Example 5. Subsequently, on embryonic day 11.5, each of 48 fetuses of wild-type mice was transplacentally transplanted with 20 ng of human cytokines (thrombopoietin, IL-6, Flt3-L, and stem cell factor) and $0.2 \times 10^4$ to $5 \times 10^4$ human cord blood-derived hematopoietic stem cells in the same manner as in Experimental Example 1. Thereafter, among the 48 mice, 34 mice were born by spontaneous delivery and allowed to grow for 4 weeks.

Then, flow cytometry analysis was performed using the peripheral blood of these mice, and the chimerism of the cells derived from the human hematopoietic stem cells was measured. The chimerism was calculated by the following Equation (3).

$$\text{Chimerism } (\%) = (\text{number of cells positive for human CD45}/(\text{number of cells positive for human CD45} + \text{number of cells positive for mouse CD45.2})) \times 100 \cdots (3)$$

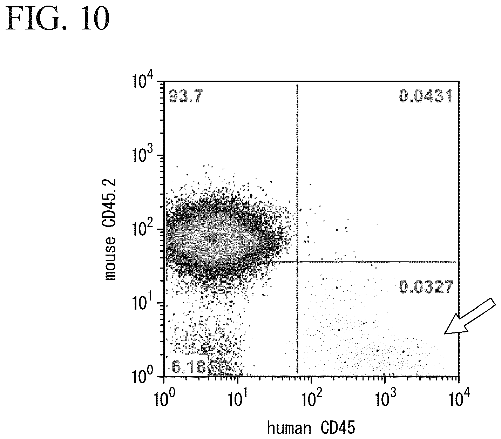

Fig. 10 shows a graph of the typical results of the flow cytometry analysis. In Fig. 10, the arrow indicates the cells positive for human CD45. As a result, it was confirmed that 0.3% or more of human-derived cells are engrafted into 3 mice among the 34 mice born.

[Experimental example 7]

(Transplantation 2 of mouse hematopoietic stem cells)

Fetuses of a mother Ly5.2 mouse having the genotype of Runx1$^{fl-}$::Tie2-Cre::G1-HRD-Runx1 (hereinafter, referred to as "Runx1 cKO mouse" in some cases) were transplacentally transplanted with hematopoietic stem cells of a C57BL/6-Ly5.1 mouse as a congenic mouse (hereinafter, referred to as "donor mouse" in some cases) in the same manner as in Experimental Example 1. Then, on embryonic day 18.5, the livers of the fetuses were collected, and the liver cells were subjected to flow cytometry analysis.

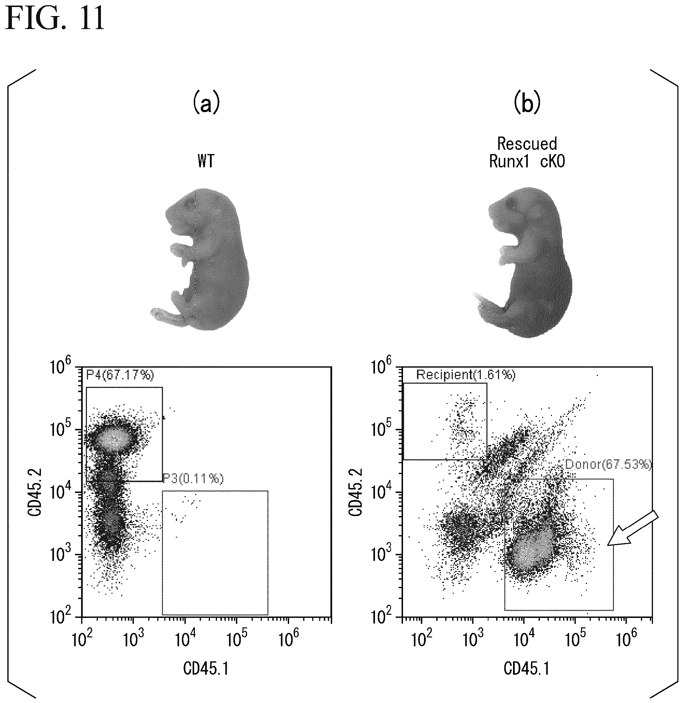

Figs. 11(a) and 11(b) show graphs of the typical results of the flow cytometry analysis. Fig. 11(a) shows the result of transplanting the hematopoietic stem cells derived from the donor mouse into fetuses of a wild-type mouse as a control. Fig. 11(b) shows the results obtained from the rescued Runx1 cKO mice. In Fig. 11(b), the arrow indicates the donor mouse-derived cells positive for CD45.1.

As a result, it was revealed that the rescued Runxl cKO mice exhibited high chimerism. Specifically, in a case where the chimerism was calculated by Equation (1) based on the results in Fig. 11(b), the chimerism (%) equaled (67.53/(67.53 + 1.61)) × 100 = 97.7%.

[Experimental example 8]

(Examination on hematopoietic reconstitution ability)

Hematopoietic stem cells derived from the Runxl cKO fetuses rescued in Experimental Example 7 were subjected to secondary transplantation, and the self-replication ability thereof was examined. Specifically, the density of liver cells of the rescued Runxl cKO fetuses on embryonic day 18.5 was adjusted to $1 \times 10^7$ cells/100 μL, and each of 6-week-old female mice having undergone total body irradiation (7 Gy) was transplanted with 100 μL of the liver cells by tail vein injection. Then, peripheral blood of the recipient mice that had undergone the secondary transplantation was analyzed by flow cytometry over time.

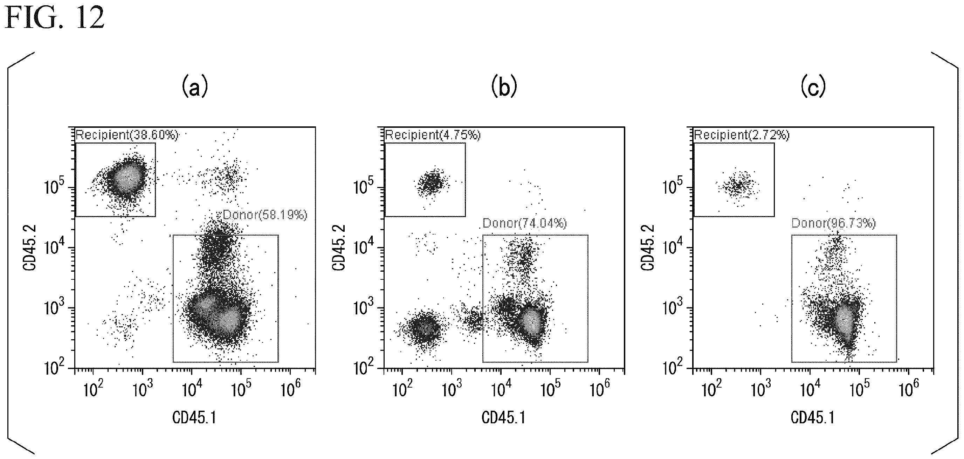

Figs. 12(a) to 12(c) show graphs of the results of the flow cytometry analysis. Fig. 12(a) shows the results obtained three weeks after the secondary transplantation, Fig. 12(b) shows the results obtained two months after the secondary transplantation, and Fig. 12(c) shows the results obtained six months after the secondary transplantation.

As a result, six months after the transplantation, the recipient mice having undergone the secondary transplantation showed high chimerism of 97.3%. This result shows that the hematopoietic stem cells derived from the rescued Runxl cKO fetuses have the hematopoietic constitution ability.

[Experimental example 9]

(Detection of human IgG in serum of mouse having undergone transplantation)

The detection of human IgG in the serum of mice transplanted with human hematopoietic stem cells was performed. Specifically, for the fetuses on embryonic day 18.5 of the Runx1$^{-/-}$::Tg mice rescued by the human hematopoietic stem cells in Experimental Example 5, human IgG in their serum was subjected to SDS-polyacrylamide gel electrophoresis, transferred to the PVDF membrane, and detected by Western blotting. Furthermore, for comparison, human IgG in the serum of the fetuses on embryonic day 18.5 of the wild-type mouse transplanted with the human hematopoietic stem cells in Experimental Example 6, and human IgG in the human serum were also subjected to detection.

For the detection of human IgG, as a primary antibody, an anti-human IgG antibody (catalog number "#62-8411", Thermo Fisher Scientific) was used. In addition, as a secondary antibody, an HRP-labeled rabbit anti-goat antibody was used.

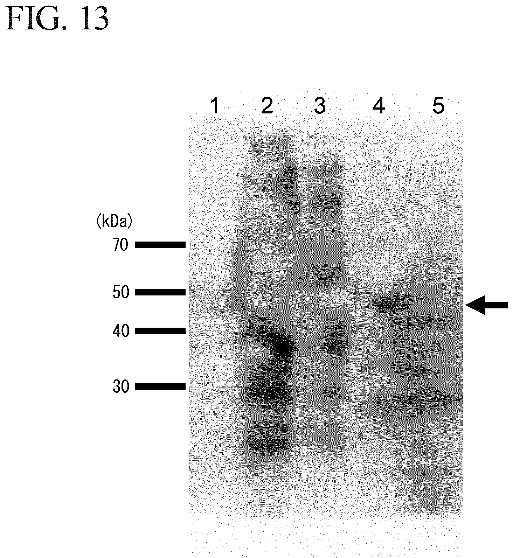

Fig. 13 is a photograph showing the results of Western blotting. In Fig. 13, lane 1 shows the result obtained from a negative control to which only a buffer was applied, lane 2 shows the result of applying a 10X diluted human serum, lane 3 shows the result of applying a 50X diluted human serum, lane 4 shows the results of applying the serum of the Runx1$^{-/-}$::Tg mice rescued by the human hematopoietic stem cells, and lane 5 shows the results of applying the serum of the wild-type mice transplanted with the human hematopoietic stem cells. Furthermore, the arrow indicates the position where a human IgG band of about 50 kDa appeared.

As a result, a human IgG band was detected in lane 4. On the other hand, no human IgG band was detected in lane 5. In addition, as a result of applying the human sera in lanes 2 and 3, the signal of 50 kDa was too strong and detected as a white blank.

From the above results, it was confirmed that human IgG is present in the serum of the Runx1$^{-/-}$::Tg mice rescued by human hematopoietic stem cells.

Industrial Applicability

According to the present invention, it is possible to provide a new technique for producing a non-human animal in which a first genetic background of blood cells is different from a second genetic background of cells other than blood cells.

**Claims**

1. A non-human animal,
   wherein blood cells have a first genetic background,
   cells other than blood cells have a second genetic background,
   the first genetic background is different from the second genetic background,
   and
   the second genetic background is a genetic mutation that does not form hematopoietic stem cells.

2. The non-human animal according to claim 1,
   wherein substantially all blood cells have the first genetic background.

3. The non-human animal according to claim 1 or 2,
   wherein the second genetic background includes knockout of Runxl gene or myb gene, conditional knockout of Runxl gene or myb gene specific to hematopoietic stem cells, or conditional knockout of Runxl gene or myb gene specific to tissue involved in the development of hematopoietic stem cells.

4. The non-human animal according to any one of claims 1 to 3,
   wherein blood cells are human cells.

5. The non-human animal according to any one of claims 1 to 3,
   wherein blood cells are rat cells.

6. A method for producing a human antibody specific to an antigen, comprising:
   a step of immunizing the non-human animal according to claim 4 with the antigen.

7. A method for producing a rat antibody specific to an antigen, comprising:
   a step of immunizing the non-human animal according to claim 5 with the antigen.

8. A method for producing human blood cells, comprising:
   a step of collecting blood cells from the non-human animal according to claim 4.

9. A method for producing a non-human animal in which a first genetic background of blood cells is different from a second genetic background of cells other than blood cells, the method comprising:

   a step of transplacentally transplanting hematopoietic stem cells having a first genetic background into an early embryo of a non-human animal having a second genetic background; and
   a step of growing the early embryo so as to obtain a non-human animal having hematopoietic stem cells,
   wherein the second genetic background is a genetic mutation that does not form hematopoietic stem cells, and the first genetic background is different from the second genetic background.

10. The method for producing according to claim 9,
    wherein the second genetic background includes knockout of Runxl gene or myb gene, conditional knockout of Runx1 gene or myb gene specific to hematopoietic stem cells, or conditional knockout of Runx1 gene or myb gene specific to tissue involved in the development of hematopoietic stem cells.

**11.** The method for producing according to claim 9 or 10, wherein the hematopoietic stem cells are human cells.

**12.** The method for producing according to claim 9 or 10, wherein the hematopoietic stem cells are rat cells.

# FIG. 1

# FIG. 2

(a) Runx1$^{+/+}$::Tg

(b) Runx1$^{-/-}$::Tg

(c) Rescued Runx1$^{-/-}$::Tg

(d) Donor:mouse

CD45.2 (vertical axis), CD45.1 (horizontal axis)

(a) 83.6, 0
(b) 44.3, 0
(c) 5.2, 82.2

(d) CHIMERISM (%), WT, Runx1$^{-/-}$::Tg

FIG. 3

FIG. 4

FIG. 5

FIG. 6

(a)           (b)

WT           Rescued
Runx1$^{-/-}$::Tg
(rat)

# FIG. 7

(a)

(b)

Donor:rat

FIG. 8

EP 3 712 266 A1

# FIG. 9

(a)

Control

mouse CD45

mouse(22.96%)

human(0.00%)

human CD45

(b)

Rescued Runx1-/-::Tg

mouse CD45

mouse(0.18%)

human(0.05%)

human CD45

FIG. 10

# FIG. 11

(a)

WT

(b)

Rescued
Runx1 cKO

FIG. 12

# FIG. 13

## INTERNATIONAL SEARCH REPORT

| International application No. |
| --- |
| PCT/JP2018/042635 |

### A. CLASSIFICATION OF SUBJECT MATTER

Int.Cl.  12N15/09(2006.01)i,  A01K67/027(2006.01)i,  C07K16/46(2006.01)i,
C12N5/078(2010.01)i

According to International Patent Classification (IPC) or to both national classification and IPC

### B. FIELDS SEARCHED

Minimum documentation searched (classification system followed by classification symbols)
Int.Cl. C12N15/09, A01K67/027, C07K16/46, C12N5/078

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
JSTPlus/JMEDPlus/JST7580 (JDreamIII), CAplus/MEDLINE/EMBASE/BIOSIS (STN),
WPIDS/WPIX (STN), AGRICOLA (STN), FSTA (STN), TOXCENTER (STN)

### C. DOCUMENTS CONSIDERED TO BE RELEVANT

| Category* | Citation of document, with indication, where appropriate, of the relevant passages | Relevant to claim No. |
| --- | --- | --- |
| A | JP 2005-229802 A (JAPAN SCIENCE AND TECHNOLOGY CORP.) 02 September 2005 & WO 2001/058255 A1 | 1-12 |
| A | JP 2012-531896 A (CHEN, Qingfeng) 13 December 2012 & US 2012/0157667 A1 & WO 2011/002727 A1 & EP 2448967 A1 | 1-12 |
| A | JP 2013-506433 A (REGENERON PHARMACEUTICALS, INC.) 28 February 2013 & JP 2013-506433 A & US 2011/0200982 A1 & WO 2011/044050 A2 & EP 2485583 A2 | 1-12 |

☒ Further documents are listed in the continuation of Box C. ☐ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 30 January 2019 (30.01.2019) | 12 February 2019 (12.02.2019) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

## INTERNATIONAL SEARCH REPORT

| | International application No. |
|---|---|
| | PCT/JP2018/042635 |

**C (Continuation).    DOCUMENTS CONSIDERED TO BE RELEVANT**

| Category* | Citation of document, with indication, where appropriate, of the relevant passages | Relevant to claim No. |
|---|---|---|
| A | WO 2016/208532 A1 (NATIONAL FEDERATION OF AGRICULTURAL COOPERATIVE ASSOCIATIONS) 29 December 2016 & US 2018/0177164 A1 & EP 3311660 A1 | 1-12 |
| A | WO 2017/038958 A1 (JICHI MEDICAL UNIVERSITY) 09 March 2017 (Family: none) | 1-12 |
| A | WILSON, E. M. et al., "Extensive double humanization of both liver and hematopoiesis in FRGN mice", Stem Cell Research, 2014, vol. 13, pp. 404-412 | 1-12 |
| A | HUANG, G. et al., "PU.1 is a major downstream target of AML1 (RUNX1) in adult mouse hematopoiesis", NATURE GENETICS, 11 November 2007, vol. 40, no. 1, pp. 51-60 | 1-12 |
| P, X | 全孝静，他，経胎盤造血幹細胞移植系を用いた血液キメラマウスの作製，第65回日本実験動物学会総会講演要旨集，27 April 2018, p. 126, O-14, (JEON, Hyojung et al.,, "Generation of reconstituted hemato-lymphoid murine embryo by intra-placental injection", Lecture abstracts of 65th annual meeting of Japanese Association for Laboratory Animal Science) | 1-12 |
| P, X | 全孝静，他，経胎盤造血幹細胞移植系を用いた血液キメラマウスの作製，第41回日本分子生物学会年会要旨，09 November 2018, 2P-0458, (JEON, Hyojung et al., "Generation of reconstituted hemato-lymphoid murine embryo by intra-placental injection", Abstracts of the 41st Anual meeting of the Molecular Biology Society of Japan) | 1-12 |
| P, X | HAMANAKA, S. et al., "Generation of Vascular Endothelial Cells and Hematopoietic Cells by Blastocyst Complementation", Stem Cell Reports, 09 October 2018, vol. 11, pp. 988-997 | 1, 2, 4-9, 11, 12 |
| P, A | | 3, 10 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017222215 A

**Non-patent literature cited in the description**

- **YOKOMIZO T. et al.** Characterization of GATA-1 + hemangioblastic cells in the mouse embryo. *The EMBO Journal,* 2007, vol. 26, 184-196
- **KISANUKI Y.Y.** Tie2-Cre Transgenic Mice: A New Model for Endothelial Cell-Lineage Analysis in Vivo. *Developmental Biology,* 2001, vol. 230, 230-242
- **BOITANO, A. E et al.** Aryl hydrocarbon receptor antagonists promote the expansion of human hematopoietic stem cells. *Science,* 2010, vol. 329 (5997), 1345-1348
- **WAGNER, JE, JR. et al.** Phase I/II Trial of StemRegenin-1 Expanded Umbilical Cord Blood Hematopoietic Stem Cells Supports Testing as a Stand-Alone Graft. *Stem Cell,* 2016, vol. 18 (1), 144-155